# EUROPEAN PATENT SPECIFICATION

(11) **EP 3 389 680 B1**
(45) Date of publication and mention of the grant of the patent: **11.10.2023**
(21) Application number: 16812728.0
(22) Date of filing: 14.12.2016
(51) Int. Cl.: A61K 35/74, A61P 1/16

(54) **TREATMENT OF INTRAHEPATIC CHOLESTASIS AND RELATED LIVER DISEASES**
BEHANDLUNG VON INTRAHEPATISCHER CHOLESTASE UND VERWANDTER LEBERERKRANKUNGEN
TRAITEMENT DE LA CHOLOSTASE INTRAHÉPATIQUE ET DE MALADIES HÉPATIQUES ASSOCIÉES

(30) Priority: 14.12.2015 US 201562266771 P
(43) Date of publication of application: 24.10.2018
(73) Proprietor: Metabogen AB, 411 26 Gothenburg (SE)
(72) Inventor: KARLSSON, Fredrik, 431 63 Molndal (SE); MÖLLSTAM, Bo, 443 31 Lerum (SE)
(74) Representative: Dehns
(86) International application number: PCT/EP2016/080941
(87) International publication number: WO 2017/102816

(56) References cited:
- WO-A1-2007/093619
- WO-A1-2015/051323
- WO-A2-00/71138
- DIYA ADAWI ET AL: "Modulation of the Colonic Bacterial Flora Affects Differently Bacterial Translocation and Liver Injury in an Acute Liver Injury Model", MICROBIAL ECOLOGY IN HEALTH & DISEASE, vol. 11, no. 1, 1 January 1999 (1999-01-01), pages 47-54, XP055342779, DOI: 10.3402/mehd.v11i1.7879
- LEPERCQ PASCALE: "Epimerization of chenodeoxycholic acid to ursodeoxycholic acid by clostridium", FEMS MICROBIOLOGY LETTERS, WILEY-BLACKWELL PUBLISHING LTD, GB, vol. 235, no. 1, 1 June 2004 (2004-06-01), pages 65-72, XP009193539, ISSN: 0378-1097

## Description

### FIELD OF THE INVENTION

The present invention relates generally to medicine. More specifically, the invention relates to products for use in probiotic interventions in mammals at risk for developing or having intrahepatic cholestasis of pregnancy.

### BACKGROUND OF THE INVENTION

Intrahepatic Cholestasis of Pregnancy (ICP) is a disease that appears in the later stage of pregnancy with itching (pruritus) and fetal complications. It is the most prevalent pregnancy-specific liver disease and is associated with an increased risk of adverse fetal outcomes, including preterm labor and intrauterine death. Further, ICP is associated with an increased risk for pre-eclampsia, thyroid disease, diabetes and cancer. The following factors may increase a woman's risk of developing ICP:
- A close relative who had ICP.
- Experienced ICP in previous pregnancies.
- Being pregnant with twins, triplets etc. (multiple pregnancy)
- Having a history of liver damage.
- In-vitro fertilization (IVF) treatment.

The diagnostic criteria for ICP have varied over time and have included clinical jaundice, severity of pruritus and elevated bile acid levels. Today, the most appropriate laboratory parameter for diagnosis of ICP is elevation of bile acids (≥ 10 µmol/L) in combination with unexplained pruritus.

Adawi et al. 1999 (Microbial Ecology in Health and Disease, 11:1, 47-54) asserts that modulation of the colonic bacterial flora affects differently bacterial translocation and liver injury in an acute liver injury model.

WO 00/71138 relates to the oral administration of Lactobacillus for the maintenance of health in women.

WO 2007/093619 relates to the use of Bifidobacterium longum for the prevention and treatment of inflammation.

WO 2015/051323 relates to compositions comprising a defined microbiome and methods of use thereof.

Lepercq et al. 2004 (FEMS microbiology letters, 235(1), 65-72) relates to epimerization of chenodeoxycholic acid to ursodeoxycholic acid by Clostridium baratii isolated from human feces.

An available pharmacologic treatment for patients with ICP is ursodeoxycholic acid (UDCA). In a double-blind, placebo-controlled trial for women with ICP, the effects of UDCA treatment was studied. 130 women were randomized to UDCA treatment, dexamethasone treatment or placebo treatment. Pruritus and biochemical markers of cholestasis were analyzed at inclusion and after 3 weeks of treatment. Also fetal complications were registered. UDCA treatment improved some biochemical markers of ICP irrespective of disease severity, but a significant relief from pruritus and marked reduction of serum bile acids were only found in patients with severe ICP (bile acid levels ≥ 40 µmol/L) and therefore there is a need for a more effective treatment. The present invention is intended to solve this problem.

### SUMMARY OF THE INVENTION

Embodiments of the invention are set out in the appended set of claims.

In particular, the present invention provides a composition comprising bile acid deconjugation activity and 7-epimerization activity, for use in a method of treating or reducing the risk of intrahepatic cholestasis in a subject, wherein said intrahepatic cholestasis comprises intrahepatic cholestasis of pregnancy, and wherein:
(i) the composition comprises at least two bacterial strains comprising
   (a) a first bacterial strain comprising bile acid deconjugation activity, and
   (b) a second bacterial strain comprising 7-epimerization activity; or
(ii) the composition comprises at least three bacterial strains comprising
   (a) a first bacterial strain comprising bile acid deconjugation activity,
   (b) a second bacterial strain comprising 7α-hydroxysteroid dehydrogenase (HSDH) activity, and
   (c) a third bacterial strain comprising 7β-HSDH activity.

The present invention relates to compositions for use in probiotic interventions in mammals at risk for developing or having intrahepatic cholestasis, wherein said intrahepatic cholestasis comprises intrahepatic cholestasis of pregnancy.

The present invention relates to compositions for use in probiotic interventions in mammals based on certain bacteria with desirable characteristics.

The composition for use of the invention may be used for modulating serum bile acid level in a mammal at risk for developing or having intrahepatic cholestasis of pregnancy.

The composition for use of the invention may be used for reducing pruritus in a mammal having intrahepatic cholestasis of pregnancy. The composition for use of the invention may be used in a method for preventing pruritus in a mammal at risk for developing intrahepatic cholestasis of pregnancy.

The composition for use of the invention may be used in methods for reducing and/or preventing the risk for the fetus in mammals at risk for developing or having intrahepatic cholestasis of pregnancy.

A primary object of the invention is to provide bacteria, e.g. probiotic bacteria, with the capability of bile acid deconjugation/hydrolysis and 7-epimerisation for use in the treatment of intrahepatic cholestasis of pregnancy. Two or more different bacterial strains are used that together exert said capabilities. As epimerization includes two reactions, oxidation of the 7α-hydroxyl group by a 7α-hydroxysteroid dehydrogenase (HSDH) and stereospecific reduction of the 7-keto functionality by a 7β-HSDH, it is another object of the invention, to choose two different probiotic bacterial strains or species, one comprising 7α-HSDH enzyme activity and the other one comprising 7β-HSDH enzyme activity to obtain the 7-epimerization reaction. In a further object of the invention the composition for use of the invention further comprises a sulphate reducing probiotic bacteria. In some embodiments, the invention comprises several bacterial strains for use of the invention that together exert the capabilities described above.

The vast majority of naturally occurring bile salts are conjugated, most commonly to glycine or taurine. Such conjugated bile salts when present in the small intestine are actively reabsorbed through the apical sodium-dependent bile transporter (ASBT) located in the ileum. Deconjugated bile acids are not actively reabsorbed in this way and a proportion of these will eventually be excreted in the feces. In addition, deconjugated bile acids are more hydrophobic than their conjugated counterparts, thus are less reabsorbed through the intestine, also resulting in higher excretion into the feces. In other words, bile acid deconjugation (also referred to herein as bile acid hydrolysis) reduces enterohepatic recirculation of bile acid and thereby reduces the total bile acid pool, including for example serum or plasma levels of bile acid. Glycine conjugates are generally present in a higher proportion (3: 1) to taurine conjugates in human bile. Therefore, a bacterial strain or species of the composition for use of the invention comprises hydrolyzing enzyme activity. In some embodiments, the hydrolysing enzyme or bile acid deconjugation activity of the bacterial strain may have a higher affinity for glycine conjugates than e.g. taurine conjugates. Thus, whilst not wishing to be bound by theory, it is believed that it is the ability of bacterial strains to achieve or stimulate bile acid deconjugation which results in reduced reabsorption or increased excretion of said bile acids, which in turn results in reduced serum levels of bile acids. Strains with this activity thus find therapeutic utility in diseases or disorders characterised by increased serum bile acid levels.

In some aspects, the hydrolysing bacterial strain (or strain comprising bile acid deconjugation activity) may comprise a strain of *Bifidobacterium,* for example *Bifidobacterium longum,* for example *Bifidobacterium longum* ATCC BAA-999.

A hydrolysing bifidobacteria (or other bacterial species or strain comprising bile acid deconjugation/hydrolysis activity as described elsewhere herein) of the composition for use of the invention may be administered to a mammal together with a prebiotic, for example which enhances the activity of the bifidobacteria (or other bacteria). A prebiotic can be, but is not limited to, inulin, starch, fructooligosaccharides (FOS) or galactooligosaccharides (GOS).

Ursodeoxycholic acid (UDCA) has been used for the treatment of ICP for some time. Absorption of ursodeoxycholic acid is slow and incomplete due to its poor solubility and it competitively inhibits the absorption of other bile acids. Several species of bacteria can convert chenodeoxycholic acid to ursodeoxycholic acid through a 7-epimerizing reaction. So instead of the addition of ursodeoxycholic acid we utilize the bacteria's enzyme activity and capability to shift the composition of the bile acids. This will for example lead to a reduced amount of bile acids in the blood stream. Also reduced levels of lithocholic acid may be produced. Epimerization includes two reactions, oxidation of the 7α-hydroxyl group by a 7α-hydroxysteroid dehydrogenase (HSDH) and stereospecific reduction of the 7-keto functionality by a 7β-HSDH.

Bacterial strains having 7-epimerizing capabilities include but are not limited to such bacteria as *Clostridium,* e.g. *Clostridium baratii,* e.g. *Clostridium baratii* ATCC 27638. In another aspect of the invention a second and a third bacterial species may be chosen, one capable of stereospecific reduction of the 7-keto functionality by a 7β-HSDH and the other having 7α-HSDH enzyme activity. Non-limiting examples of probiotic bacterial strains having 7α-HSDH enzyme activity include species of *Bacteroides,* for example *Bacteroides fragilis* (e.g. *Bacteroides fragilis* ATCC 25285) and *Bacteroides thetaiotaomicron* (e.g. *Bacteroides thetaiotaomicron* DSM 2079) and a non-limiting example of a strain having 7β-HSDH enzyme activity include species of *Collinsella,* for example *Collinsella aerofaciens* (e.g. *Collinsella aerofaciens* ATCC 25986).

As more bile acids may be in a deconjugated form due to administration of a hydrolysing bacterial strain, an increase in free taurine concentration may take place. Therefore, in a further object of the invention, the composition for use further comprises a sulfate reducing bacteria, thereby avoiding the detrimental effects of the increase in taurine. Such sulfate reducing bacteria include, but are not limited to species of *Desulfovibrio,* for example *Desulfovibriopiger* (e.g. *Desulfovibrio piger* DSM 32187). In some embodiments, the use of such sulfate reducing bacteria is preferred.

### DETAILED DESCRIPTION OF THE INVENTION

The present invention thus relates generally to a composition of probiotic bacterial strains for use in a method of treating or preventing (reducing the risk of) intrahepatic cholestasis, wherein said intrahepatic cholestasis comprises intrahepatic cholestasis of pregnancy. Such treatments are based on the selection of two or more bacterial strains (or species) to provide a composition comprising bile acid deconjugation (or hydrolysis) activity and 7-epimerization activity. The two or more strains for use in the therapeutic methods will have both bile acid deconjugation and 7-epimerization activity, provided by way of a combination of strains. Such two or more strains are for use in a method of treating or preventing (reducing the risk of) intrahepatic cholestasis, wherein said intrahepatic cholestasis comprises intrahepatic cholestasis of pregnancy. Such therapeutic treatments are generally based on the ability of said bacterial strains to modulate and preferably reduce serum bile acid level, e.g. modulating (e.g. reducing) bile acid level, e.g. bodily fluid bile acid level such as serum bile acid level.

The foregoing and other aspects of the present invention will now be described in more detail with respect to the description and methodologies provided herein. It should be appreciated that the invention may be embodied in different forms and should not be construed as limited to the embodiments set forth herein. Rather, these embodiments are provided so that this disclosure will be thorough and complete, and will fully convey the scope of the invention to those skilled in the art.

The terminology used in the description of the invention herein is for the purpose of describing particular embodiments only and is not intended to be limiting of the invention.

As used in the description of the embodiments of the invention, the singular forms "a," "an" and "the" are intended to include the plural forms as well, unless the context clearly indicates otherwise. Also, as used herein, "and/or" refers to and encompasses any and all possible combinations of one or more of the associated listed items.

Furthermore, the term "about," as used herein when referring to a measurable value such as an amount of a compound, dose, time, temperature, and the like, refers to variations of 20%, 10%, 5%, 1%, 0.5%, or even 0.1% of the specified amount.

When a range is employed (e.g., a range from x to y) it is it meant that the measurable value is a range from about x to about y, or any range therein, such as about x₁ to about yi, etc.

It will be further understood that the terms "comprises" and/or "comprising," when used in this specification, specify the presence of stated features, integers, steps, operations, elements, and/or components, but do not preclude the presence or addition of one or more other features, integers, steps, operations, elements, components, and/or groups thereof.

In some embodiments of the invention, the compositions for example do not contain any active ingredients or components beyond those specified, e.g. consist of those active ingredients or components. Thus, for example, where compositions comprising particular bacterial strains are described herein, in some embodiments the compositions will consist of those bacterial strains and not contain any other bacterial strains. In other embodiments such compositions will not contain any further or additional active ingredients or components.

Unless otherwise defined, all terms, including technical and scientific terms used in the description, have the same meaning as commonly understood by one of ordinary skill in the art to which this invention belongs.

"Effective amount" or dosage as used herein refers to an amount of a bacterial composition of the invention that is sufficient to produce a desired effect, which can be a therapeutic and/or beneficial effect. The effective amount or dosage will vary with the age, general condition of the subject, the severity of the condition being treated, the particular composition administered, the duration of the treatment, the nature of any concurrent treatment, the pharmaceutically acceptable carrier used, and like factors within the knowledge and expertise of those skilled in the art. As appropriate, an "effective amount" or dosage in any individual case can be determined by one of skill in the art by reference to the pertinent texts and literature and/or by using routine experimentation.

By the term "treat," "treating," or "treatment of" (and grammatical variations thereof) it is meant that the severity of the subject's condition is reduced, at least partially improved or ameliorated and/or that some alleviation, mitigation or decrease in at least one clinical symptom is achieved and/or there is a delay in the progression of the disease or disorder.

As will be clear from the disclosure elsewhere herein, the compositions for use of the prevent invention are suitable for reducing the risk of disease or prevention of disease as well as active treatment of diseases (for example treatment of pre-existing disease), wherein the disease is intrahepatic cholestasis, wherein said intrahepatic cholestasis comprises intrahepatic cholestasis of pregnancy. Thus, prophylactic treatment is also encompassed by the invention.

Such preventative (or protective) aspects can conveniently be carried out on healthy or normal or at risk subjects and can include both complete prevention and significant prevention. Similarly, significant prevention can include the scenario where severity of disease or symptoms of disease is reduced (e.g. measurably or significantly reduced) compared to the severity or symptoms which would be expected if no treatment is given.

A "therapeutically effective" amount as used herein is an amount that is sufficient to treat (as defined herein) the subject. Those skilled in the art will appreciate that the therapeutic effects need not be complete or curative, as long as some benefit is provided to the subj ect.

As used herein, the terms "modulating," "modulate," "modulates" or grammatical variations thereof, means an alteration, for example in the amount or level of a component in a bodily fluid (e.g., blood, serum, and the like) of a subject, by administering to the subject a therapeutically effective amount of a composition comprising at least two bacterial, e.g. probiotic bacterial, species or strains, thereby increasing or reducing the amount or level of the component. In embodiments of the invention where bodily fluid, e.g. blood or serum, bile acid levels are modulated, then preferably the amount or level of bile acid is reduced or decreased.

The terms "increase," "increasing," "increased," "higher", "enhance," "enhanced," "enhancing," and "enhancement" (and grammatical variations thereof), as used herein, describe an elevation, for example, an elevation in the amount or level of a component in a bodily fluid (e.g., blood, serum, and the like) of a subject (e.g., an elevation of at least about 20%, 25%, 30%, 35%, 40%, 45%, 50%, 75%, 100%, 125%, 150%, 175%, 200%, 350%, 300%, 350%, 400%, 450%, 500% or more). This increase in expression, amount or level, can be observed by, for example, comparing the amount or level of a component in a bodily fluid of a subject to the amount or level of the same component in the bodily fluid of a control subject that, for example, has not been administered a therapeutically effective amount of a composition comprising at least two bacterial, e.g. probiotic bacterial, species or strains as described herein.

As used herein, the terms "reduce," "reduced," "reducing," "reduction," "diminish," "suppress," and "decrease" (and grammatical variations thereof), describe, for example, a decrease in the amount or level of a component in a bodily fluid (e.g., blood, serum, and the like) of a subject, e.g.as compared to a control as described herein. Thus, this decrease in amount or level can be observed by, for example, comparing the amount or level of a component in a bodily fluid of a subject to the amount or level of the same component in the bodily fluid of a control subject that, for example, has not been administered a therapeutically effective amount of a composition comprising at least two probiotic bacterial species or strains as described herein. In preferred embodiments of the invention, decreases in bile acid levels in a body fluid of a subject (e.g. serum bile acid levels), e.g. compared to a control subject, are obtained.

Appropriate control subjects would readily be identified by a person skilled in the art and might include a non-treated or placebo-treated subject (or a population thereof), or might include a level of a particular parameter, e.g. bile acid level, in the same individual subject measured at an earlier time point, such as before treatment (e.g. comparison with a "baseline" level in that subject). Preferably the increase or decrease (as appropriate) will be significant, for example clinically or statistically significant, for example with a probability value of ≤0.05, when compared to an appropriate control level or value.

A "subject" of the invention includes any animal that has or is susceptible to intrahepatic cholestasis (or any other disease or disorder for treatment by the present invention as described elsewhere herein). In some embodiments, the subject can be a mammal. Mammalian subjects include but are not limited to humans, non-human primates (*e.g.*, gorilla, monkey, baboon, and chimpanzee, etc.), dogs, cats, goats, horses, pigs, cattle, sheep, and the like, and laboratory animals (e.g., rats, guinea pigs, mice, gerbils, hamsters, and the like). Suitable subjects include both males and females and subjects of any age, including embryonic (*e.g.*, *in utero* or *in ovo or fetal*), infant, juvenile, adolescent, adult and geriatric subjects. In some embodiments, a subject of this invention is a human. In representative embodiments, the subject is a human female. In other representative embodiments, the subject is a pregnant human female, and/or her fetus or unborn baby which can suffer adverse effects from intrahepatic cholestasis (or other diseases or disorders as described herein).

A "subject" or a "subject in need" of the methods of the invention can be a subject known to have or suspected of having or at risk of developing intrahepatic cholestasis, wherein said intrahepatic cholestasis comprises cholestasis of pregnancy. In representative embodiments, a subject or subject in need can be a subject known to have or suspected of having or at risk of developing intrahepatic cholestasis of pregnancy (ICP). A subject or subject in need thereof may also be a subject known to have, suspected of having, or at risk of developing pruritus (in particular pruritus arising as a symptom of ICP). In some embodiments, a subject or subject in need can be a subject that is known to have, is suspected of having, or is at risk of developing gall stones, biliary dyspepsia, primary biliary cirrhosis, primary sclerosing cholangitis, chronic active hepatitis, hepatitis C and/or other related liver diseases.

The compositions for use of the invention can also be used to treat, alleviate (reduce or relieve) or prevent (reduce the risk of) symptoms or side effects of intrahepatic cholestasis of pregnancy, for example pruritus.

In preferred embodiments, a subject or subject in need is a subject known to have, or suspected of having, or at risk of developing intrahepatic cholestasis, in particular intrahepatic cholestasis of pregnancy. The methods of the invention can be used to treat or reduce the risk of intrahepatic cholestasis of pregnancy associated with or characterised by altered and preferably increased bile acid level, in particular serum bile acid level, e.g. serum bile acid levels of ≥ 10 µmol/L (e.g. 10 to 39 µmol/L), or ≥ 40 µmol/L (e.g. 40 to 79 µmol/L), or ≥ 80 µmol/L. In some embodiments, subjects with serum bile acid levels of ≥ 10 µmol/L and < 40 µmol/L are preferred.

As used herein the term "concomitant administration" or "combination administration" of a composition, therapeutic agent or known drug (e.g., prebiotic) with a bacterial composition for use of the present invention means administration of a known medication or drug and, in addition, the two or more bacterial species or strains of the composition for use of the invention at such time that both the known drug and the bacterial species or strains will have a therapeutic effect. In some cases, this therapeutic effect will be synergistic. Such concomitant administration can involve concurrent (i.e., at the same time, in parallel at the same time), prior, or subsequent administration (e.g., sequential) of the known drug with respect to the administration of a bacterial composition for use of the present invention. Such concomitant or combination administration may also refer to administration of a compound (e.g. bacterial strains or species) for use of the invention through different administrative routes separately (e.g., at least about 2 or more hours apart), sequentially (e.g., within about 2 hours, e.g., about15 sec, 30 sec, 45 sec, 1 min, 2, min, 3 min, 4 min, 5 min, 6 min, 7 min, 8 min, 9 min, 10 min, 11 min, 12 min, 13 min, 14 min, 15 min, 16 min, 17 min, 18 min, 19 min, 20 min, 21 min, 22 min, 23 min, 24 min, 25 min, 26 min, 27 min, 28 min, 29 min, 30 min, 35 min, 40 min, 45 min, 50 min, 55 min, 60 min, 65 min, 70 min, 75 min, 80 min, 85 min, 90 min, 95 min, 100 min, 105 min, 110 min, 115 min, and the like, and any range or value therein) and/or in parallel at the same time (e.g., concurrently) in order to achieve effective amount or dosage. A person of skill in the art, would have no difficulty determining the appropriate timing, sequence and dosages of administration for particular drugs and bacterial compositions for use of the present invention.

"Probiotics" are live microorganisms that, when administered in adequate amounts, confer a health benefit to the host.

Intrahepatic cholestasis of pregnancy (ICP) usually occurs during the last trimester of pregnancy. It may trigger severe pruritus, especially on the hands and feet (often without rash), but is rarely of concern for the mother's long-term health. However it may cause severe complications for the fetus such as pre-term (premature) birth, fetal distress, inhaling meconium during birth resulting in breathing difficulties (e.g. meconium aspiration syndrome) and also intrauterine death or stillbirth. Thus, the methods and uses of the invention can treat or reduce the risk of such complications in the fetus as well as treating or reducing the risk of ICP and symptoms thereof in the mother. Women that previously have experienced ICP have a higher risk for developing ICP during their later pregnancies; also women with relatives that have had ICP have an increased risk for developing ICP. Other risk factors associated with developing ICP are multiple pregnancies, a history of liver damage and in-vitro fertilization (IVF) treatment. Such women are further examples of subjects or subjects in need (e.g. at risk subjects) that can be treated in accordance with the present invention.

The diagnostic criteria for ICP have varied over time and have included clinical jaundice, severity of pruritus and elevated bile acid levels, generally in the blood, e.g. serum or plasma. Today, the most appropriate laboratory parameter for diagnosis of ICP is elevation of bile acids (≥ 10 µmol/L) in combination with unexplained pruritus. The incidence of ICP was found to be 1.5 % in a prospective cohort study in Sweden. A mild form of ICP (81% of ICP patients) can be defined as bile acid levels < 40 µmol/L (but ≥ 10 µmol/L) and a severe form of ICP (19% of ICP patients) defined as bile acid levels ≥ 40 µmol/L, which is also associated with a higher rate of fetal complications. The probability of fetal complications increased by 1% -2% per µmol/L of total bile acid increase. Any forms of ICP can be treated with the methods of the present invention. For example, in a preferred embodiment, mild forms of ICP as defined above are treated. In other embodiments severe forms of ICP as defined above are treated.

In the last trimester, the pregnancy hormones, particularly progesterone, cause muscular tissue to relax, which will prepare the birth canal prior to delivery. This can also affect the proper function of the gallbladder, which has the purpose of storing bile.

Bile acids/salts play a critical role in activating digestive enzymes and solubilizing fats and fat-soluble vitamins and are involved in liver, biliary, and intestinal disease. Bile acids are synthesized in the liver by a multistep, multiorganelle pathway. Bile salts are excreted by the hepatocytes into the canaliculi to form bile. The canaliculi drain into the right and left hepatic ducts and the bile flows to the gallbladder. Bile is released from the gallbladder and travels to the duodenum, where it contributes to the metabolism and degradation of fat.

The common bile acids differ primarily in the number and orientation of hydroxyl groups on the sterol ring. The term, primary bile acid refers to those synthesized *de novo* by the liver. In humans, the primary bile acids include cholic acid (3α, 7α, 12α-trihydroxy-5β-cholanic acid) ("CA") and chenodeoxycholic acid (3α, 7α-dihydroxy-5β-cholanic acid) ("CDCA"). Dehydroxylation of these bile acids by intestinal bacteria produces the more hydrophobic secondary bile acids, deoxycholic acid (3α, 12α-dihydroxy-5β-cholanic acid) ("DCA") and lithocholic acid (3α-hydroxy-5β-cholanic acid) ("LCA"). These four bile acids CA, CDCA, DCA, and LCA, generally constitute greater than 99 percent of the bile salt pool in humans. Secondary bile acids that have been metabolized by the liver are sometimes denoted as tertiary bile acids.

Keto-bile acids are produced secondarily in humans as a consequence of oxidation of bile acid hydroxyl groups, particularly the 7-hydroxyl group, by colonic bacteria. However, keto-bile acids are rapidly reduced by the liver to the corresponding α or β -hydroxy bile acids. For example, the corresponding keto bile acid of a CDCA is 7-keto lithocholic acid and one of its reduction products with the corresponding beta-hydroxy bile acid is ursodeoxycholic acid (3α-7β-dihydroxy-5β-cholanic acid) ("UDCA"), a tertiary bile acid.

UDCA, a major component of bear bile, has been used as a pharmaceutical agent for the treatment of and the protection against many types of liver disease for a little over 70 years. Its medicinal uses include the dissolution of radiolucent gall stones, the treatment of biliary dyspepsia, primarily biliary cirrhosis, primary sclerosing cholangitis, chronic active hepatitis and hepatitis C. In other mammalian species, bile acids containing a 6β-hydroxyl group, which are found in rats and mice, are known as muricholic acid, and 6α-hydroxy bile acids produced by swine are termed hyocholic acid and hyodeoxycholic acids. 23-hydroxy bile acids of aquatic mammals are known as phocecholic and phocedeoxycholic acids.

Typically, more than 99 percent of naturally occurring bile salts secreted into human bile are conjugated. Conjugates are bile acids in which a second organic substituent (e.g. glycine, taurine, glucuronate, sulfate or, rarely, other substituents) is attached to the side chain carboxylic acid or to one of the ring hydroxyl groups via an ester, ether, or amide linkage. Conjugated bile acids are less cytotoxic than free bile acids.

Around 95% of bile salts that are released into the small intestine are actively reabsorbed. Bile salt reabsorption, in particular of the conjugated bile acids, occurs via the apical sodium-dependent bile transporter (ASBT) present in the brush border membrane of the enterocyte. Once bile salts reach the basolateral membrane they are transported into the blood. A small percentage of the bile salts are not actively reabsorbed and undergo deconjugation by the intestinal microbiota before either being absorbed or converted into secondary bile acids, deoxycholate (DCA, from cholate) and lithocholate and ursodeoxycholate (LCA and UDCA, from chenodeoxycholate). Secondary bile acids are either passively absorbed or are excreted in the feces. The absorbed primary and secondary bile acids and salts are transported back to the liver where most, but not all, are actively transported into hepatocytes. Once in the liver the bile acids are reconjugated and then resecreted together with newly synthesized bile salts. This overall process constitutes one cycle of what is called the enterohepatic circulation. Within the intestinal lumen, bile acid concentrations vary, with the bulk of the reuptake occurring in the distal intestine. Bile acids/salts alter the growth of bacterial flora in the gut. The bile acid pool contains about 2-4 g of bile acids and this pool is recycled via the enterohepatic circulation on the order of six to ten times each day. Of the total bile salt pool, around 0.2-0.6 g are excreted in the feces each day. This lost fraction of bile salts is replenished via de novo hepatic bile acid synthesis from cholesterol.

The present invention is based on the principle that combinations of certain bacteria could decrease the serum bile acid level and relieve pruritus in women suffering from intrahepatic cholestasis of pregnancy. Thus, the present invention relates to compositions for use for probiotic interventions in mammals at risk for developing or having or suspected of having intrahepatic cholestasis, wherein the intrahepatic cholestasis comprises intrahepatic cholestasis of pregnancy.

The compositions for use of the invention may modulate (e.g. reduce) serum bile acid level and treat or reduce pruritus or the risk of pruritus in a subject, e.g. mammal, having intrahepatic cholestasis wherein the mammals or subjects are at risk for developing or having or suspected of having intrahepatic cholestasis of pregnancy.

The composition for use of the invention comprising probiotic bacteria having the capability of bile acid deconjugation/hydrolysis and 7-epimerisation is for use in the treatment of intrahepatic cholestasis of pregnancy. The composition for use comprises a combination of several bacterial strains or species (i.e., more than one, e.g., 2, 3, 4, 5, 6 and the like bacterial species or strains) that together exert or provide the capabilities described above.

As described elsewhere herein, deconjugated bile acids are not actively reabsorbed by the ASBT and a proportion of these will eventually be excreted in the faeces. In addition, deconjugated bile acids are more hydrophobic than their conjugated counterparts, thus are less reabsorbed through the intestine, also resulting in higher excretion into the feces. In other words, bile acid deconjugation reduces enterohepatic recirculation of bile acid, thereby reducing the total bile acid pool. Absorption of ursodeoxycholic acid (UDCA) is slow and incomplete due to poor solubility and it competitively inhibits the absorption of other bile acids. Several species of bacteria can convert chenodeoxycholic acid to ursodeoxycholic acid through a 7-epimerizing reaction. Epimerization includes two reactions, oxidation of the 7α-hydroxyl group by a 7α-hydroxysteroid dehydrogenase (HSDH) and stereospecific reduction of the 7-keto functionality by a 7β-HSDH.

In one embodiment, a combination of probiotic bacteria strains can comprise one strain with capability of bile acid deconjugation/hydrolysis and another strain having the capability of 7-epimerisation. In some embodiments, the hydrolysing bacterial strain can comprise *Bifidobacterium,* e.g. *Bifidobacterium longum,* e.g. *Bifidobacterium longum* ATCC BAA-999. Glycine conjugates are generally higher in proportion (3: 1) to taurine conjugates in human bile; therefore, in some embodiments, the bile acid deconjugation/hydrolysis activity of the bacterial strain may have a higher affinity or activity for glycine conjugates, e.g. than taurine conjugates. In some embodiments, a bacterial strain having 7-epimerizing enzyme activity can comprise *Clostridium,* e.g. *Clostridium baratii,* e.g. *Clostridium baratii* ATCC 27638.

In another embodiment, said probiotic bacterial strain combination can comprise one strain and/or species comprising the enzyme activity of bile acid deconjugation/hydrolysis and more than one strain and/or species to provide the 7-epimerizing enzyme activity, for example another strain and/or species comprising the enzyme activity of 7α-HSDH and yet another strain and/or species comprising the enzyme activity of 7β-HSDH. Epimerization of bile acid hydroxyl groups is the reversible change in stereochemistry from α to β configuration (or vise versa). In the context of the present invention, 7-epimerization activity can result in the reversible change of CDCA to UDCA. The extent of the reversible oxidation and reduction of bile acid hydroxyl groups by HSDH depends in part on the redox potential of the environment in the gut. Higher redox potential, favoring oxidation by the 7α-HSDH, is found on the mucosal surface and a lower redox potential, favoring reduction by the 7β-HDSH, is found in the intestinal lumen. When using two different strains (one with 7α-HSDH enzyme activity and the other one with 7β-HDSH enzyme activity) for obtaining the 7-epimerizing reaction the advantage of using the varying redox potential is utilized. In some embodiments, a hydrolysing bacterial strain/species can comprise *Bifidobacterium,* e.g. *Bifidobacterium longum,* e.g. *Bifidobacterium longum* ATCC BAA-999. In some embodiments, exemplary bacterial species or strains having 7α-HSDH enzyme activity can include *Bacteroides,* e.g. *Bacteroides fragilis,* e.g *Bacteroides fragilis* ATCC 25285 and *Bacteroides thetaiotaomicron,* e.g. *Bacteroides thetaiotaomicron* DSM 2079. *Collinsella,* e.g. *Collinsella aerofaciens,* e.g. *Collinsella aerofaciens* ATCC 25986, is a nonlimiting example of a strain or species with 7β-HSDH enzyme activity.

In some embodiments, as more bile acids may be in a deconjugated form, due to administration of a hydrolysing bacterial strain/species, an increase in free taurine concentration may possibly take place. Some pathogenic bacteria have the unique capability of metabolizing taurine to hydrogen sulfide, H₂S, which is highly toxic. Thus, in a further embodiment of the invention, the composition for use of the invention may further comprise a sulfate reducing bacteria to outcompete the metabolic activity of such pathogenic bacteria, thus avoiding negative effects of the released taurine. *Desulfovibrio,* e.g. *Desulfovibrio piger,* e.g. *Desulfovibrio piger* DSM 32187, is one example of a strain with sulfate reducing capabilities.

In one embodiment of the invention, an example of a probiotic bacteria strain/species combination can be *Bifidobacterium, Bacteroides* and *Collinsella, or Bifidobacterium* and *Clostridium.* Examples of appropriate bacteria include one or more or all of (e.g. 2 or 3 of) *Bifidobacterium longum, Bacteroides fragilis,* and *Collinsella aerofaciens,* or one or more or all of (e.g. 2 or 3 of) *Bifidobacterium longum, Bacteroides thetaiotaomicron,* and *Collinsella aerofaciens.* Examples of appropriate bacteria also include one or both of *Bifidobacterium longum* and *Clostridium baratii.* Any of these combinations may further comprise *Desulfovibrio* bacteria, e.g. *Desulfovibrio piger.*

Preferred examples of strains to be used in the above combinations are one or more or all of (e.g. 2 or 3 or 4 of) *Bifidobacterium longum* ATCC BAA-999, *Bacteroides fragilis* ATCC 25285, *Collinsella aerofaciens* ATCC 25986, *Bacteroides thetaiotaomicron* DSM 2079, *Clostridium baratii* ATCC 27638, or *Desulfovibrio piger* DSM 32187.

In one embodiment of the invention, an example of a probiotic bacteria strain/species combination can be *Bifidobacterium longum* ATCC BAA-999, *Bacteroides fragilis* ATCC 25285, *Collinsella aerofaciens* ATCC 25986 and *Desulfovibriopiger* DSM 32187. In further embodiment of the invention, an example of a probiotic bacterial combination comprises *Bifidobacterium longum* ATCC BAA-999, *Bacteroides thetaiotaomicron* DSM 2079, *Collinsella aerofaciens* ATCC 25986 and *Desulfovibriopiger* DSM 32187. In yet another embodiment of the invention, a probiotic bacteria combination comprises *Bifidobacterium longum* ATCC BAA-999, *Clostridium baratii* ATCC 27638 and *Desulfovibrio piger* DSM 32187. Other examples would be these combinations without *Desulfovibrio piger* DSM 32187.

Compositions for use comprising the above combinations of bacterial strains or species provide a yet further aspect of the invention. Such compositions may take the form of pharmaceutical compositions or nutritional compositions. Some exemplary compositions are provided in the Examples.

The bifidobacteria (or other bacteria as discussed above) of the composition for use of the invention may be administered to a mammal together with a prebiotic which may enhance the activity of the bifidobacteria (or other bacteria). A prebiotic can include inulin. Other examples of prebiotics are described elsewhere herein.

In one embodiment of the invention, a probiotic bacteria strain combination as described herein can be for use in mammals (or subjects) at risk for developing ICP. Risk factors include previous experience of ICP, relatives that have had ICP (i.e. genetic factors), multiple pregnancies, history of liver damage and in-vitro fertilization (IVF) treatment. In some embodiments, the administration can be about one, two or three times/day and treatment may start when the mammal becomes pregnant. The administration may also start when the mammal is planning to get pregnant, if said mammal is at risk of developing ICP. In another embodiment, the administration can be about one, two or three times/day and treatment may start in the second trimester (around week 13 of pregnancy).

In another embodiment of the invention, a probiotic bacteria strain combination as described herein can be for use in a mammal (or subject) having ICP. In some embodiments, the treatment group comprises a mammal having bile acids levels that exceed 10 µmol/L (or 40 µmol/L or 80 µmol/L) and unexplained pruritus, or patients with mild or severe ICP as described elsewhere herein. In some embodiments, the administration can be one, two or three times/day and last for one, two or three months or as long as said mammal is pregnant or until the baby is born.

In some embodiments of the invention, a probiotic bacteria strain combination as described herein can be for use in a mammal (or subject) at risk for developing or having ICP (e.g. ICP subject groups as defined elsewhere herein) in order to decrease the risk for the fetus and/or baby. The decreased risk for the fetus can include a decreased risk for pre-term birth, inhaling meconium during birth resulting in breathing difficulties and/or also intrauterine death or stillbirth.

In the invention, a probiotic bacteria strain combination as described herein is for use in intrahepatic cholestasis comprising intrahepatic cholestasis of pregnancy. In some such embodiments, the treatment group comprises a mammal having increased bile acid levels, e.g. bile acids levels, in particular serum bile acid levels, that exceed 10 µmol/L (or 40 µmol/L or 80 µmol/L), e.g. serum bile acid levels of ≥ 10 µmol/L (e.g. 10 to 39 µmol/L or ≥ 10 µmol/L and < 40 µmol/L), or ≥ 40 µmol/L (e.g. 40 to 79 µmol/L or ≥ 40 µmol/L and < 80 µmol/L), or ≥ 80 µmol/L.

Preferred products or compositions for use as described herein comprise frozen, freeze-dried, lyophilized (e.g. lyophilized powder), or dried bacteria and are preferably in a unit-dosage format, e.g. a capsule (e.g. gelatin capsule) or tablet or gel or sachet. Appropriate ratios and doses (e.g. in the form of numbers of bacteria or CFUs) for use in such products, etc., are described elsewhere herein and in the Examples. Other components may also be included in such products for use, etc., for example preservatives (e.g. glycerol), dessicants, stabilizers, gelling agents and/or cryoprotectants. In some embodiments such additional components are non-natural agents. In some embodiments a prebiotic will be included. Examples of prebiotics are described elsewhere herein.

In one embodiment of the invention, a probiotic bacteria strain combination described herein can be in the form of a capsule or a sachet or other suitable forms and can be orally administered to the mammal (or subject). In some embodiments, the administration can be one, two or three times/day and last for one, two or three months or as long as said mammal continues to suffer from the disease being treated.

Appropriate doses of the species or strains for use in the invention can be chosen depending on the disease to be treated, the mode of administration and the formulation concerned. For example, a dosage and administration regime is chosen such that the probiotic combination of bacteria administered to the subject in accordance with the present invention can result in a therapeutic or health benefit. An appropriate dose of each bacteria is selected such that a therapeutic or health benefit is observed when all strains are present. For example, daily doses of one or each bacteria of 10⁴ to 10¹², for example 10⁵ to 10¹⁰, or 10⁶ to 10⁸, or 10⁷ to 10⁹,or 10⁸ to 10¹⁰ total CFUs of bacteria may be used. A preferred daily dose of one or each bacteria is around 10⁸ or 10⁹ total CFUs, e.g. 10⁷ to 10¹⁰ or 10⁸ to 10¹⁰ or 10⁸ to 10⁹. Such doses can be in the form of CFU/g or CFU/unit-dosage form (e.g. per sachet, capsule, tablet, etc).

Exemplary regimes are provided in the Examples.

The uses of the invention can be carried out on any animal or subject or patient. In some embodiments, an animal or subject can be a mammal. In representative embodiments, the animal or subject can be a human.

Thus, a composition for use in a method of treating or reducing the risk of intrahepatic cholestasis in a subject in need thereof, wherein said intrahepatic cholestasis comprises intrahepatic cholestasis of pregnancy, is provided, comprising administering to the subject a therapeutically effective amount of a composition comprising at least two bacterial species or strains comprising bile acid deconjugation/hydrolysis activity and 7-epimerization activity, thereby treating or reducing the risk of intrahepatic cholestasis in a subject in need thereof.

Thus, the present invention provides a composition comprising at least two bacterial species or strains comprising bile acid deconjugation/hydrolysis activity and 7-epimerization activity, for use in a method of treating or reducing the risk of intrahepatic cholestasis in a subject, wherein said intrahepatic cholestasis comprises intrahepatic cholestasis of pregnancy.

The composition for use may modulate bile acid level, e.g. serum bile acid level, in a subject in need thereof. In particular embodiments, the subject has or is at risk of developing cholestasis, intrahepatic cholestasis (e.g., intrahepatic cholestasis of pregnancy), gall stones, the treatment of biliary dyspepsia, primary biliary cirrhosis, primary sclerosing cholangitis, chronic active hepatitis, hepatitis C, and/or other related liver diseases.

In preferred embodiments, such modulation or modulating is by way of a reduction or decrease in bile acid level.

The use of the composition for use of the invention may additionally comprise treating or reducing the risk of developing gall stones, the treatment of biliary dyspepsia, primary biliary cirrhosis, primary sclerosing cholangitis, chronic active hepatitis, hepatitis C, and/or other related liver diseases, in a subject.

In embodiments, the use of the composition for use of the invention may be in a method of treating or reducing the risk of pruritis in a subject. In particular embodiments, the subject has or is at risk of developing cholestasis, intrahepatic cholestasis (e.g., intrahepatic cholestasis of pregnancy), gall stones, the treatment of biliary dyspepsia, primary biliary cirrhosis, primary sclerosing cholangitis, chronic active hepatitis, hepatitis C, and/or other related liver diseases.

Such pruritus can be associated with intrahepatic cholestasis comprising intrahepatic cholestasis of pregnancy.

In some embodiments, intrahepatic cholestasis comprises intrahepatic cholestasis of pregnancy.

In some embodiments, the subject is a mammal. In some embodiments, the subject is a human. In representative embodiments, the subject is a human female, for example a pregnant human female.

Any appropriate bacterial species or strain, for example any appropriate probiotic bacterial species or strain, can be used in the methods or uses of the present invention, providing they have the functional properties as described herein.

At least two bacterial species or strains in combination are used in the methods and uses of the present invention (for example 2, 3, 4, or 5 species or strains may be used). The strains can be of the same or different species of bacteria. Equally, more than one species of bacteria can be used in certain embodiments.

A combination or mixture of bacterial strains or species are administered (combination therapy). Such mixtures or combinations of bacteria can be administered together in a single (the same) composition or administered separately (e.g. in different products or compositions). If administered separately then such administration may be sequential or simultaneous. However, the separate administration forms part of the same therapeutic regimen or method.

In embodiments where the administration of the two or more strains or species is separate or sequential, it is preferred that the administrations are made within a reasonable time frame of each other as described elsewhere herein. For example, the separate administrations are preferably made within hours (e.g. one hour) or minutes (e.g. within 15 or 30 minutes) of each other, most preferably within as short a timeframe as possible (including simultaneous or effectively simultaneous administration). Preferably the two or more strains or species of bacteria are co-administered in a single composition.

In embodiments where more than one strain of probiotic bacteria is used in a mixture in a single composition, or where more than one strain of probiotic bacteria is used but they are administered separately, then any appropriate ratio of the bacteria can be used providing that the desired function of the strains (for example bile acid deconjugation/hydrolysis activity or 7-epimerization activity or sulphate reducing activity) is retained to a useful extent. Such ratios can readily be determined by a person skilled in the art. For example, such a combination of two or more strains or species might be used at a ratio of 1:10, 1:5, 1:1, 5:1, or 10:1 or anywhere between these extremes, e.g. 1:1. Such ratios may also be used in the products, kits, compositions, etc., of the invention as described elsewhere herein.

Such species or strains are generally isolated species or strains, or pure cultures, which can then be mixed together if a combination of species or strains is used. In some embodiments such strains will not correspond to naturally occurring strains.

In some embodiments, the composition for use of the invention comprises at least two bacterial species or strains, the at least two bacterial species or strains comprising a first bacterial species or strain comprising bile acid deconjugation/hydrolysis activity and a second bacterial species or strain comprising 7-epimerization activity.

In some embodiments, the composition for use of the invention comprises at least three bacterial species or strains, the at least three bacterial species or strains comprising a first bacterial species or strain comprising bile acid deconjugation/hydrolysis activity, a second bacterial species or strain comprising 7α-hydroxysteroid dehydrogenase (HSDH) activity, and a third bacterial species or strain comprising 7β-HSDH activity.

In some embodiments, the composition may further comprise at least one bacterial species or strain comprising sulfate reducing activity. This activity may be provided by one or more of the same bacterial species or strains which provide the bile acid deconjugation or 7-epimerization activity or may be provided by at least one additional (or different) bacterial species or strain. In some embodiments, the bacterial species or strain comprising sulfate reducing activity may be *Desulfovibrio piger* DSM 32187. This strain of *Desulfovibrio piger* for use in the present invention has been deposited under the Budapest Treaty at DSMZ (Leibniz Institute DSMZ - German Collection of Microorganisms and Cell Cultures, Inhoffenstr. 7B, D-38124 Braunschweig, Germany) on October 20, 2015.

In some embodiments, the bacterial species or strain comprising the activity of bile acid deconjugation/hydrolysis activity may be a bifidobacteria. In representative embodiments, the bacterial species or strain comprising the activity of bile acid deconjugation/hydrolysis activity may be *Bifidobacterium longum* ATCC BAA-999.

In some embodiments, the bacterial species or strain comprising 7-epimerization activity may be *Clostridium baratii* ATCC 27638.

In some embodiments, the bacterial species or strain comprising 7α-HSDH activity may be *Bacteroides fragilis* ATCC 25285 and/or *Bacteroides thetaiotaomicron* DSM 2079, and/or the bacterial species or strain comprising 7β-HSDH activity may be *Collinsella aerofaciens* ATCC 25986.

In some embodiments, the composition for use of the invention comprises *Bifidobacterium longum* ATCC BAA-999, *Bacteroides fragilis* ATCC 25285, and *Collinsella aerofaciens* ATCC 25986.

In some embodiments, the composition for use of the invention comprises *Bifidobacterium longum* ATCC BAA-999, *Bacteroides thetaiotaomicron* DSM 2079, and *Collinsella aerofaciens* ATCC 25986.

In some embodiments, the composition for use of the invention comprises *Bifidobacterium longum* ATCC BAA-999 and *Clostridium baratii* ATCC 27638.

In some embodiments, the composition for use of the invention comprises *Bifidobacterium longum* ATCC BAA-999, *Bacteroides fragilis* ATCC 25285, *Collinsella aerofaciens* ATCC 25986, and *Desulfovibriopiger* DSM 32187.

In some embodiments, the composition for use of the invention comprises *Bifidobacterium longum* ATCC BAA-999, *Bacteroides thetaiotaomicron* DSM 2079, *Collinsella aerofaciens* ATCC 25986 and *Desulfovibriopiger* DSM 32187.

In some embodiments, the composition for use of the invention comprises *Bifidobacterium longum* ATCC BAA-999, *Clostridium baratii* ATCC 27638 and *Desulfovibriopiger* DSM 32187 In some embodiments, the composition for use of the invention further comprises administering to the subject a prebiotic, for example in the form of a composition comprising a therapeutically effective amount of a prebiotic. In some embodiments, the prebiotic is administered prior to, concurrently with, or after administration of the probiotic bacteria. In some embodiments, the prebiotic may be inulin. Other examples of prebiotics are described elsewhere herein.

The prebiotic may be in the same composition as the probiotic bacteria or administered separately, for example in a different composition.

### EXAMPLES

The present invention will now be described with reference to the following examples. It should be appreciated that these examples are for the purpose of illustrating aspects of the present invention.

### EXAMPLE 1

A product (Product A) is developed and contains the following bacterial strains:
1) *Bifidobacterium longum* ATCC BAA-999
*2) Clostridium baratii* ATCC 27638
*3) Desulfovibrio piger* DSM 32187

The bacterial strains in the product are in the concentration of 1^{∗}10⁸ cfu/g/bacterial strain and in the form of lyophilized powder in standard gelatin capsules of 500 mg.

### EXAMPLE 2

A product (Product B) is developed and manufactured in the form of a sachet containing a composition of:
1) *Bifidobacterium longum* ATCC BAA-999: 1^{∗}10⁸ cfu / sachet
*2) Bacteroides fragilis* ATCC 25285: 1^{∗}10⁸ cfu / sachet
*3) Collinsella aerofaciens* ATCC 25986: 1^{∗}10⁸ cfu / sachet
4) Inulin: 3000 mg / sachet

The composition is filled at ambient temperature into aluminum foil bags as known in the art with desiccant (10 cm x 12 cm, using packaging material PET12/PE/ALU 12/PE/PE+desiccant/PE from Alcan) in a LAF bench (Holten Laminair Model S-2010 1.2 from Heto-Holten A/S, Denmark). To each bag, 3 g of powder with *B. longum* ATCC BAA-999, *B. fragilis* ATCC 25285 and C. *aerofaciens* ATCC 25986 and inulin is added using the balance XP-600 from Denver Instrument GmbH, Germany. The filled aluminum foil bags are then heat sealed with the film sealing device model F460/2 from Kettenbaum Folienschweisstechnik GmbH & Co. KG, Germany.

### EXAMPLE 3

A test group of pregnant women with mild ICP (bile acid levels ≥ 10 µmol/L and < 40 µmol/L) is included in a study and receive the probiotic Product B (as described in Example 2). The treatment takes place three times/day and lasts for three months or until delivery of the baby. Prior to the treatment the women is examined by obtaining blood samples and by registering the percept severity of pruritus/itching. Blood samples is analysed with regards to concentration of bile acids.

After treatment, the women are examined once again as described above. Women that are treated with the probiotic Product B show decreased levels of bile acids in the blood and experience a relief in pruritus/itching.

## Claims

1. A composition comprising bile acid deconjugation activity and 7-epimerization activity, for use in a method of treating or reducing the risk of intrahepatic cholestasis in a subject, wherein said intrahepatic cholestasis comprises intrahepatic cholestasis of pregnancy, and wherein:
(i) the composition comprises at least two bacterial strains comprising
(a) a first bacterial strain comprising bile acid deconjugation activity, and
(b) a second bacterial strain comprising 7-epimerization activity; or
(ii) the composition comprises at least three bacterial strains comprising
(a) a first bacterial strain comprising bile acid deconjugation activity,
(b) a second bacterial strain comprising 7α-hydroxysteroid dehydrogenase (HSDH) activity, and
(c) a third bacterial strain comprising 7β-HSDH activity.

2. The composition for use of claim 1, wherein:
the first bacterial strain recited in part (i) is *Bifidobacterium*;
the second bacterial strain recited in part (i) is *Clostridium*;
the first bacterial strain recited in part (ii) is *Bifidobacterium*;
the second bacterial strain recited in part (ii) is *Bacteroides*; and/or
the third bacterial strain recited in part (ii) is *Collinsella.*

3. The composition for use of claim 2, wherein:
the first bacterial strain recited in part (i) is *Bifidobacterium longum*;
the second bacterial strain recited in part (i) is *Clostridium baratii*;
the first bacterial strain recited in part (ii) is *Bifidobacterium longum*;
the second bacterial strain recited in part (ii) is *Bacteroides fragilis* and/or *Bacteroides thetaiotaomicron*; and/or
the third bacterial strain recited in part (ii) is *Collinsella aerofaciens.*

4. The composition for use of claim 3, wherein:
the first bacterial strain recited in part (i) is *Bifidobacterium longum* ATCC BAA-999;
the second bacterial strain recited in part (i) is *Clostridium baratii* ATCC 27638;
the first bacterial strain recited in part (ii) is *Bifidobacterium longum* ATCC BAA-999;
the second bacterial strain recited in part (ii) is *Bacteroides fragilis* ATCC 25285 and/or *Bacteroides thetaiotaomicron* DSM 2079; and/or
the third bacterial strain recited in part (ii) is *Collinsella aerofaciens* ATCC 25986.

5. The composition for use of any one of claims 1 to 4, wherein the composition further comprises at least one bacterial strain comprising sulfate reducing activity.

6. The composition for use of claim 5, wherein the bacterial strain comprising sulfate reducing activity is *Desulfovibrio.*

7. The composition for use of claim 6, wherein the bacterial strain comprising sulfate reducing activity is *Desulfovibriopiger.*

8. The composition for use of claim 7, wherein the bacterial strain comprising sulfate reducing activity is *Desulfovibriopiger* DSM 32187.

9. The composition for use of any one of claims 1 to 8,
wherein the composition comprises *Bifidobacterium longum* ATCC BAA-999, *Bacteroides fragilis* ATCC 25285, and *Collinsella aerofaciens* ATCC 25986; or
wherein the composition comprises *Bifidobacterium longum* ATCC BAA-999, *Bacteroides thetaiotaomicron* DSM 2079, and *Collinsella aerofaciens* ATCC 25986; or
wherein the composition comprises *Bifidobacterium longum* ATCC BAA-999 and *Clostridium baratii* ATCC 27638; or
wherein the composition comprises *Bifidobacterium longum* ATCC BAA-999, *Bacteroides fragilis* ATCC 25285, *Collinsella aerofaciens* ATCC 25986, and *Desulfovibrio piger* DSM 32187; or
wherein the composition comprises *Bifidobacterium longum* ATCC BAA-999, *Bacteroides thetaiotaomicron* DSM 2079, *Collinsella aerofaciens* ATCC 25986 and *Desulfovibrio piger* DSM 32187; or
wherein the composition comprises *Bifidobacterium longum* ATCC BAA-999, *Clostridium baratii* ATCC 27638 and *Desulfovibriopiger* DSM 32187.

10. The composition for use of any one of claims 1 to 9, wherein said use further comprises a prebiotic.

11. The composition for use of claim 10, wherein the prebiotic is inulin.

12. The composition for use of any one of claims 1 to 11, wherein the subject is a mammal.

13. The composition for use of claim 12, wherein the subject is a human.

14. The composition for use of any one of claims 1 to 13, wherein said use comprises treating or reducing the risk of pruritis in the subject.

## Patentansprüche

1. Zusammensetzung, die Aktivität der Dekonjugation von Gallensäuren und Aktivität der 7-Epimerisierung umfasst, zur Verwendung in einem Verfahren zur Behandlung oder Reduzierung des Risikos einer intrahepatischen Cholestase in einem Subjekt, wobei die intrahepatische Cholestase eine intrahepatische Schwangerschaftscholestase umfasst, und wobei:
(i) die Zusammensetzung mindestens zwei Bakterienstämme umfasst, umfassend
(a) einen ersten Bakterienstamm, der die Aktivität der Dekonjugation von Gallensäure umfasst, und
(b) einen zweiten Bakterienstamm, der die Aktivität der 7-Epimerisierung umfasst; oder
(ii) die Zusammensetzung mindestens drei Bakterienstämme umfasst, umfassend
(a) einen ersten Bakterienstamm, der die Aktivität der Dekonjugation von Gallensäure umfasst,
(b) einen zweiten Bakterienstamm, der die Aktivität der 7α-Hydroxysteroiddehydrogenase (HSDH) umfasst, und
(c) einen dritten Bakterienstamm, der die 7β-HSDH-Aktivität umfasst.

2. Zusammensetzung zur Verwendung nach Anspruch 1, wobei:
der erste in Teil (i) genannte Bakterienstamm *Bifidobacterium* ist;
der zweite in Teil (i) genannte Bakterienstamm *Clostridium* ist;
der erste in Teil (ii) genannte Bakterienstamm *Bifidobacterium* ist;
der zweite in Teil (ii) genannte Bakterienstamm *Bacteroides* ist; und/oder
der dritte in Teil (ii) genannte Bakterienstamm *Collinsella* ist.

3. Zusammensetzung zur Verwendung nach Anspruch 2, wobei:
der erste in Teil (i) genannte Bakterienstamm *Bifidobacterium longum* ist;
der zweite in Teil (i) genannte Bakterienstamm *Clostridium baratii* ist;
der erste in Teil (ii) genannte Bakterienstamm *Bifidobacterium longum* ist;
der zweite in Teil (ii) genannte Bakterienstamm *Bacteroides fragilis* und/oder *Bacteroides thetaiotaomicron* ist; und/oder
der dritte in Teil (ii) genannte Bakterienstamm *Collinsella aerofaciens* ist.

4. Zusammensetzung zur Verwendung nach Anspruch 3, wobei:
der erste in Teil (i) genannte Bakterienstamm *Bifidobacterium longum* ATCC BAA-999 ist;
der zweite in Teil (i) genannte Bakterienstamm *Clostridium* baratii ATCC 27638 ist;
der erste in Teil (i) genannte Bakterienstamm *Bifidobacterium longum* ATCC BAA-999 ist;
der zweite in Teil (ii) genannte Bakterienstamm *Bacteroides fragilis* ATCC 25285 und/oder *Bacteroides thetaiotaomicron* DSM 2079 ist; und/oder
der dritte in Teil (ii) genannte Bakterienstamm *Collinsella aerofaciens* ATCC 25986 ist.

5. Zusammensetzung zur Verwendung nach einem der Ansprüche 1 bis 4, wobei die Zusammensetzung weiter mindestens einen Bakterienstamm umfasst, der eine sulfatreduzierende Aktivität aufweist.

6. Zusammensetzung zur Verwendung nach Anspruch 5, wobei der die sulfatreduzierende Aktivität umfassende Bakterienstamm *Desulfovibrio* ist.

7. Zusammensetzung zur Verwendung nach Anspruch 6, wobei der die sulfatreduzierende Aktivität umfassende Bakterienstamm *Desulfovibriopiger* ist.

8. Zusammensetzung zur Verwendung nach Anspruch 7, wobei der die sulfatreduzierende Aktivität umfassende Bakterienstamm *Desulfovibriopiger* DSM 32187 ist.

9. Zusammensetzung zur Verwendung nach einem der Ansprüche 1 bis 8,
wobei die Zusammensetzung *Bifidobacterium longum* ATCC BAA-999, *Bacteroides fragilis* ATCC 25285 und *Collinsella aerofaciens* ATCC 25986 umfasst; oder
wobei die Zusammensetzung *Bifidobacterium longum* ATCC BAA-999, *Bacteroides thetaiotaomicron* DSM 2079 und *Collinsella aerofaciens* ATCC 25986 umfasst; oder
wobei die Zusammensetzung *Bifidobacterium longum* ATCC BAA-999 und *Clostridium baratii* ATCC 27638 umfasst; oder
wobei die Zusammensetzung *Bifidobacterium longum* ATCC BAA-999, *Bacteroides fragilis* ATCC 25285, *Collinsella aerofaciens* ATCC 25986, und *Desulfovibriopiger* DSM 32187 umfasst; oder
wobei die Zusammensetzung *Bifidobacterium longum* ATCC BAA-999, *Bacteroides thetaiotaomicron* DSM 2079, *Collinsella aerofaciens* ATCC 25986 und *Desulfovibriopiger* DSM 32187 umfasst; oder
wobei die Zusammensetzung *Bifidobacterium longum* ATCC BAA-999, *Clostridium baratii* ATCC 27638 und *Desulfovibriopiger* DSM 32187 umfasst.

10. Zusammensetzung zur Verwendung nach einem der Ansprüche 1 bis 9, wobei die Verwendung weiter ein Präbiotikum umfasst.

11. Zusammensetzung zur Verwendung nach Anspruch 10, wobei das Präbiotikum Inulin ist.

12. Zusammensetzung zur Verwendung nach einem der Ansprüche 1 bis 11, wobei das Subjekt ein Säugetier ist.

13. Zusammensetzung zur Verwendung nach Anspruch 12, wobei das Subjekt ein Mensch ist.

14. Zusammensetzung zur Verwendung nach einem der Ansprüche 1 bis 13, wobei die Verwendung die Behandlung oder Reduzierung des Risikos von Pruritis in dem Subjekt umfasst.

## Revendications

1. Composition comprenant une activité de déconjugaison des acides biliaires et une activité de 7-épimérisation, pour une utilisation dans un procédé de traitement ou de réduction du risque de cholestase intrahépatique chez un sujet, dans laquelle ladite cholestase intrahépatique comprend la cholestase intrahépatique de la grossesse, et dans laquelle :
(i) la composition comprend au moins deux souches bactériennes comprenant
(a) une première souche bactérienne comprenant une activité de déconjugaison des acides biliaires, et
(b) une deuxième souche bactérienne comprenant une activité de 7-épimérisation ; ou
(ii) la composition comprend au moins trois souches bactériennes comprenant
(a) une première souche bactérienne comprenant une activité de déconjugaison des acides biliaires,
(b) une deuxième souche bactérienne comprenant une activité 7α-hydroxystéroïde déshydrogénase (HSDH), et
(c) une troisième souche bactérienne comprenant une activité 7β-HSDH.

2. Composition pour une utilisation selon la revendication 1, dans laquelle :
la première souche bactérienne mentionnée dans la partie (i) est *Bifidobacterium* ;
la deuxième souche bactérienne mentionnée dans la partie (i) est *Clostridium* ;
la première souche bactérienne mentionnée dans la partie (ii) est *Bifidobacterium* ;
la deuxième souche bactérienne mentionnée dans la partie (ii) est *Bacteroides* ; et/ou
la troisième souche bactérienne mentionnée dans la partie (ii) est *Collinsella.*

3. Composition pour une utilisation selon la revendication 2, dans laquelle :
la première souche bactérienne mentionnée dans la partie (i) est *Bifidobacterium longum* ;
la deuxième souche bactérienne mentionnée dans la partie (i) est *Clostridium baratii* ;
la première souche bactérienne mentionnée dans la partie (ii) est *Bifidobacterium longum* ;
la deuxième souche bactérienne mentionnée dans la partie (ii) est *Bacteroides fragilis* et/ou *Bacteroides thetaiotaomicron* ; et/ou
la troisième souche bactérienne mentionnée dans la partie (ii) est *Collinsella aerofaciens.*

4. Composition pour une utilisation selon la revendication 3, dans laquelle :
la première souche bactérienne mentionnée dans la partie (i) est *Bifidobacterium longum* ATCC BAA-999 ;
la deuxième souche bactérienne mentionnée dans la partie (i) est *Clostridium baratii* ATCC 27638 ;
la première souche bactérienne mentionnée dans la partie (ii) est *Bifidobacterium longum* ATCC BAA-999 ;
la deuxième souche bactérienne mentionnée dans la partie (ii) est *Bacteroides fragilis* ATCC 25285 et/ou *Bacteroides thetaiotaomicron* DSM 2079; et/ou
la troisième souche bactérienne mentionnée dans la partie (ii) est *Collinsella aerofaciens* ATCC 25986.

5. Composition pour une utilisation selon l'une quelconque des revendications 1 à 4, dans laquelle la composition comprend en outre au moins une souche bactérienne comprenant une activité de réduction des sulfates.

6. Composition pour une utilisation selon la revendication 5, dans laquelle la souche bactérienne comprenant une activité de réduction des sulfates est *Desulfovibrio.*

7. Composition pour une utilisation selon la revendication 6, dans laquelle la souche bactérienne comprenant une activité de réduction des sulfates est *Desulfovibrio piger.*

8. Composition pour une utilisation selon la revendication 7, dans laquelle la souche bactérienne comprenant une activité de réduction des sulfates est *Desulfovibrio piger* DSM 32187.

9. Composition pour une utilisation selon l'une quelconque des revendications 1 à 8,
dans laquelle la composition comprend *Bifidobacterium longum* ATCC BAA-999, *Bacteroides fragilis* ATCC 25285 et *Collinsella aerofaciens* ATCC 25986 ; ou
dans laquelle la composition comprend *Bifidobacterium longum* ATCC BAA-999, *Bacteroides thetaiotaomicron* DSM 2079 et *Collinsella aerofaciens* ATCC 25986 ; ou
dans laquelle la composition comprend *Bifidobacterium longum* ATCC BAA-999 et *Clostridium baratii* ATCC 27638 ; ou
dans laquelle la composition comprend *Bifidobacterium longum* ATCC BAA-999, *Bacteroides fragilis* ATCC 25285, *Collinsella* aerofaciens ATCC 25986 et *Desulfovibrio piger* DSM 32187 ; ou
dans laquelle la composition comprend *Bifidobacterium longum* ATCC BAA-999, *Bacteroides thetaiotaomicron* DSM 2079, *Collinsella aerofaciens* ATCC 25986 et *Desulfovibrio piger* DSM 32187 ; ou
dans laquelle la composition comprend *Bifidobacterium longum* ATCC BAA-999, *Clostridium baratii* ATCC 27638 et *Desulfovibrio piger* DSM 32187.

10. Composition pour une utilisation selon l'une quelconque des revendications 1 à 9, dans laquelle ladite utilisation comprend en outre un prébiotique.

11. Composition pour une utilisation selon la revendication 10, dans laquelle le prébiotique est l'inuline.

12. Composition pour une utilisation selon l'une quelconque des revendications 1 à 11, dans laquelle le sujet est un mammifère.

13. Composition pour une utilisation selon la revendication 12, dans laquelle le sujet est un humain.

14. Composition pour une utilisation selon l'une quelconque des revendications 1 à 13, dans laquelle ladite utilisation comprend le traitement ou la réduction du risque de prurit chez le sujet.
